# EUROPEAN PATENT APPLICATION

(11) **EP 1 787 679 A1**
(43) Date of publication of application: **23.05.2007**
(21) Application number: 05384031.0
(22) Date of filing: 29.07.2005
(51) Int. Cl.: A61P 25/02

(54) **Use of compounds binding to the sigma receptor for the treatment of diabetes-associated pain**

(71) Applicant: LABORATORIOS DEL DR. ESTEVE, S.A., 08041 Barcelona (ES)
(72) Inventor: Buschman, Helmut Heinrich, Barcelona (ES)
(74) Representative: Peters, Hajo

(57) **Abstract**

The present invention refers to the use of compounds active on the sigma receptor for the production of a medicament for the treatment of diabetes-associated pain.

## Description

### Field of the invention

The present invention refers to the use of compounds binding to the sigma receptor for the production of a medicament for the treatment of diabetes-associated pain, as well as the prevention or the prophylaxis of the symptoms of diabetes-associated pain.

### Background of the invention

Diabetes is a metabolic disorder caused by interaction of genetic, enviromental, immunological, as well as life-style factors. In 2004, according to the World Health Organization (WHO), more than 150 million people worldwide suffer from diabetes. Its incidence is increasing rapidly and it is estimated that by the year 2025 this number will double. According to the American Diabetes Association (ADA; http://www.diabetes.org/home.jsp) has identified four major categories of diabetes including:
Type 1 diabetes mellitus: The body's fails to produce insulin
Type 2 diabetes mellitus: Results from insulin resistance, combined with relative insulin deficiency
Gestational diabetes: Occurs during pregnancy.
Impaired glucose tolerance (i.e. prediabetes): When a person's blood glucose levels are higher than normal but not high enough for a diagnosis of type 2 diabetes.

In the pathological course of diabetes often further complications may arise such as periperal vascular disease, diabetic neuropathy, diabetic foot problems, diabetic retinopathy and nephropathy. At least some of these complications may cause light, moderate or severe pain symptoms which represents a big problem for the many patients suffering from this disease.

This problem was solved by the present invention which relates to new ways for the treatment of diabetes-associated pain.

Thus, the present invention relates to the new use of compounds binding to the sigma receptor for the production of a medicament for the treatment of diabetes-associated pain, preferably diabetes-associated neuropathic pain, as well as the prevention or the prophylaxis of the symptoms of diabetes-associated pain.

This/these compound/s are preferably in neutral form, the form of a base or acid, in the form of a salt, preferably a physiologically acceptable salt, in the form of a solvate or of a polymorph and/or in the form of in the form of its racemate, pure stereoisomers, especially enantiomers or diastereomers or in the form of mixtures of stereoisomers, especially enantiomers or diastereomers, and/or in any mixing ratio.

"The sigma receptor/s" as used in this application is/are well known and defined using the following citation: This binding site represents a typical protein different from opioid, NMDA, dopaminergic, and other known neurotransmitter or hormone receptor families (G. Ronsisvalle et al. Pure Appl. Chem. 73, 1499-1509 (2001)). Pharmacological data based on ligand binding studies, anatomical distribution and biochemical features distinguish at least two subtypes of σ receptors (R. Quiron et al., Trends Pharmacol. Sci. 13, 85-86 (1992); M.L.Leitner, Eur. J. Pharmacol. 259, 65-69 (1994); S.B. Hellewell and W.D. Bowen; Brain Res. 527, 244-253 (1990)) (G. Ronsisvalle et al. Pure Appl. Chem. 73, 1499-1509 (2001)). The protein sequence of sigma receptors (Sigma 1 (σ1) and Sigma 2 (σ2)) is known (e.g. Prasad, P.D. et al., J. Neurochem. 70 (2), 443-451 (1998)) and they show a very high affinity for e.g. pentazocine.

"Compound/s binding to the sigma receptor" as used in this application is/are defined as having an IC₅₀ value ≤5000 nM, more preferably ≤1000 nM, more preferably ≤500 nM, more preferably ≤250 nM, more preferably ≤100 nM. Most preferably, the IC₅₀ value is ≤50 nM. Additionally, the wording "Compound/s binding to the sigma receptor", as used in the present application is defined as having at least ≥ 50% displacement using 10 mM radioligand specific for the sigma receptor (e.g. preferably ¹H-pentazocine) whereby the sigma recepor may be any sigma receptor subtype.

Preferably, said compounds binding to the sigma receptor are specific for the sigma-1 receptor.

In another possible embodiment of the present invention said compounds binding to the sigma receptor may be specific for the sigma-2 receptor.

Compounds binding to the sigma receptor, generally also known as sigma ligands, are well known in the art and many of them are falling under the definition for "Compound/s binding to the sigma receptor" set up above.

Preferably, Ifenprodil may optionally be disclaimed from the present invention.

Preferably, compounds selected from the group consisting of Amitriptyline, Clomipramine, Desipramine, Duloxetine, Fenfluramine, Fluoxetine, Fluphenazine, Maprotiline, Methadone, Mexiletine, Nortriptyline are disclaimed from the present invention. The majority of these compounds has an IC₅₀ ≥100 nM with respect to binding the sigma receptor.

Preferably, compounds selected from the group consisting of ABT-594, Acarbose, alpha-lipoic acid, amantadine, Aspirin, Azathioprine, Benfotiamine, BT-594, Capsaicin, Carbamazepine, Cl-988, Clonidine, Cyclophosphamide, Gabapentin, Insulin, JTT-501, Lamotrigine, Lidocaine, Metoclopramide, MK-801, Morphine, Neurotropin, Nimodipine, OT-7100, Oxycarbazepine, Pamidronate, Pentoxifylline, Pindolol, Prednisone, RP-67580, Sarpogrelate, SD-282, SR-48,968, Sulindac, Tebonin, Topiramate, Venlafaxine, WIN 55,212-2, Zonisamide. The majority of these compounds has an IC₅₀ ≥ 100 nM with respect to binding the sigma receptor.

Compounds binding to the sigma receptor known in the art and matching the criteria of sigma ligand (i.e. having an IC₅₀ ≤5000 nM) as mentioned above, are listed below. Some of these compounds may bind to the sigma-1 and/or the sigma-2 receptor. Preferably, these compounds are in form of a salt, a base or an acid. Also preferably, the salts/bases/acids indicated in the list are to be understood as being exemplary and therefore may respresent any salt, base or acid of the compound.

| | |
|---|---|
| (-)-Cyanopindolol hemifumarate | (-)-SPARTEINE SULFATE PENTAHYDRATE |
| (+)-HIMBACINE | (2-Dibutylamino-Ethyl)-Carbamic Acid 2-(4-Benzofuran-2-Ylmethyl-Piperazin-1-Yl)-Ethyl Ester |
| (4-[1,2,3]Thiadiazol-4-Yl-Benzyl)-Carbamic Acid 1-(3-Methoxy-2-Nitro-Benzyl)-Piperidin-3-Ylmethyl Ester | (S)-Methamphetamine HCl |
| [1-(9-Ethyl-9H-Carbazol-3-Ylmethyl)-Pyrrolidin-3-Yl]-Carbamic Acid 1-(3-Benzyloxy-4-Methoxy-Benzyl)-Piperidin-3-Ylmethyl Ester | [1-(9-Ethyl-9H-Carbazol-3-Ylmethyl)-Pyrrolidin-3-Yl]-Carbamic Acid 2-(Tert-Butoxycarbonyl-Naphthalen-1-Ylmethyl-Amino)-Ethyl Ester |
| [4-(4-Ethyl-3,5-Dimethyl-Pyrazol-1-Yl)-Phenyl]-[4-(3-Phenyl-Allyl)-Piperazin-1-Yl]-Methanone | 1-(1,2-Diphenylethyl)Piperidine Maleate, (+/-) |
| 1-(1-Naphthyl)Piperazine HCl | 1-(3-Chlorophenyl)Piperazine HCl |
| 1-(4-Bromo-Benzenesulfonyl)-4-(2-Tert-Butylsulfany-Benzyl)-Piperazine | 2-(2-{[1-(3-Chloro-Benzyl)-Pyrrolidin-3-Yl]-Methyl-Carbamoyl}-2-Methyl-Propyl)-4,6-Dimethyl-Benzoic Acid |
| 2-Chloro-11-(4-Methylpiperazino)Dibenz[B,F]Oxepin Maleate | 3,3'-Diethylthiacarbocyanine lodide |
| 3-Mercapto-2-Methylpropanoic Acid 1,2-Diphenylethylamine Salt | 3-Quinuclidinyl Benzilate |
| 3-Tropanyl-3,5-Dichlorobenzoate | 3-Tropanyl-Indole-3-Carboxylate HCl |
| 4-(1H-Indol-4-Yl)-Piperazine-1-Carboxylic Acid 2-(5-Bromo-2-Ethoxy-Phenylamino)-Cyclohexylmethyl Ester | 4-(2-Tert-Butylsulfanyl-Benzyl)-Piperazine-1-Carboxylic Acid 2-Thiophen-2-Yl-Ethyl Ester |
| 4-(3,5-Dimethoxy-Phenyl)-Piperazine-1-Carboxylic Acid 1-(2-Fluoro-Benzyl)-Piperidin-2-Ylmethyl Ester | 4-(3-Nitro-5-Sulfamoyl-Thiophen-2-Yl)-Piperazine-1-Carboxylic Acid 1-(2-Fluoro-5-Methoxy-Benzyl)-Piperidin-3-Ylmethyl Ester |
| 4-(4-Fluorobenzoyl)-1-(4-Phenylbutyl)Piperidine Oxalate | 4-(5-Trifluoromethyl-Pyridin-2-Yl)-Piperazine-1-Carboxylic Acid Pent-2-Ynyl Ester |
| 4,4'-Bis[4-(P-Chlorophenyl)-4-Hydroxypiperidino]Butyrophenone | 4-[1-(4-Chlorobenzyl)-4-(benzylpiperidin-4-yl]-2-hydroxy-4-oxobut-2-enoic acid |
| 4-Bromo-N-[1-(9-Ethyl-9H-Carbazol-3-Ylmethyl)-Pyrrolidin-3-Yl]-2-Trifluoromethoxy-Benzenesulfonamide | 4'-Chloro-3-Alpha-(Diphenylmethoxy)Tropane HCl |
| 4-Furan-2-Ylmethyl-Piperazine-1-Carboxylic Acid 2-{4-[3-(2-Trifluoromethyl-Phenothiazin-10-Yl)- Propyl]-Piperazin-1-Yl}-Ethyl Ester | 4-Methoxy-N-[1-(7-Methoxy-Benzo[1,3]Dioxol-5-Ylmethyl)-Pyrrolidin-3-Yl]-Benzenesulfonamide |
| 5-(N-Ethyl-N-Isopropyl)-Amiloride | 7-Hydroxy-DPAT HBr, (±)- |
| 8-Hydroxy-DPAT HBr, (R)-(+)- | 8-Hydroxy-DPAT HBr, S(-)- |
| 9-[4-({[4'-(trifluoromethyl)-1,1'-biphenyl-2-yl]carbonyl}amino)piperidin-1-yl]-N-(2,2,2-trifluoroethyl)-9H-fluorene-9-carboxamide | Acepromazine Maleate |
| Acetophenazine Maleate | Acrinol |
| Ajmaline | Alaproclate HCl |
| Aloe-Emodin | Alprenolol D-Tartrate Salt Hydrate |
| Alprenolol HCl | AMI-193 |
| Aminobenztropine | Amiodarone HCl |
| Amodiaquine HCl | Amorolfine HCl |
| Amoxapine | Anileridine HCl |
| Anisotropine Methylbromide | Anpirtoline |
| ARC 239 DiHCl | Astemizole |
| Auramine O HCl | Azaperone |
| Azatadine Maleate | Azelastine HCl |
| Bamethan sulfate | BD 1008 DiHBr |
| BD-1047 (N1-(3,4-dichlorophenethyl)-N1,N2,N2-trimethylethane-1,2-diamine) | BD-1063 (1-(3,4-dichlorophenethyl)-4-methylpiperazine). |
| Benextramine TetraHCl | Benfluorex HCl |
| Benidipine HCl | Benoxathian HCl |
| Benoxinate HCl | Benperidol |
| Benproperine Phosphate | Benzododecinium bromide |
| Benzphetamine HCl | Benztropine Mesylate |
| Benzydamine HCl | Bephenium Hydroxynaphthoate |
| Bepridil HCl | Berberine chloride |
| Betaxolol HCl | Bifemelane |
| BMY 7378 DiHCl | Bopindolol Malonate |
| BP 554 Maleate | Bromhexine HCl |
| Bromodiphenhydramine HCl | Bromperidol |
| Brompheniramine Maleate | BTCP HCl |
| Buclizine HCl | Buflomedil HCl |
| Bupropion HCl | Buspirone HCl |
| Butacaine Sulfate | Butaclamol HCl, (±)- |
| Butenafine HCl | Butoconazole Nitrate |
| BW 723C86 HCl | Carbetapentane Citrate |
| Carbinoxamine Maleate | Carpipramine DiHCl DiH2O |
| Carvedilol | Cephapirin Benzathine |
| CGS-12066A Maleate | Chloroprocaine HCl |
| Chloroquine Phosphate | Chlorpheniramine Maleate |
| Chlorphenoxamine HCl | Chlorpromazine HCl |
| Chlorprothixene | Cinanserin HCl |
| Cinnarizine | Cirazoline HCl |
| Cis-(+/-)-N-Methyl-N-[2-(3,4-Dichlorophenyl)Ethyl]-2-( 1-Pyrrolidinyl)Cyclohexamine DiHBr | Cis(Z)-Flupentixol DiHCl |
| Cisapride Hydrate | Citalopram HBr |
| Clebopride Maleate Salt | Clemastine Fumarate |
| Clemizole HCl | Clenbuterol HCl |
| Clidinium Bromide | Clobenpropit 2HBr |
| Clofazimine | Clofilium Tosylate |
| Clomiphene Citrate | Clomiphene Related Compound A |
| Cloperastine HCl | Clorgyline HCl |
| Clozapine | CONESSINE |
| Cyclizine | Cyclobenzaprine HCl |
| Cycloheximide | Cyproheptadine HCl |
| Darrow Red HCl | Demecarium Bromide |
| Denatonium Benzoate | Deptropine Citrate |
| Desloratadine | Dexbrompheniramine Maleate |
| Dexchlorpheniramine Maleate | Dexfenfluramine HCl |
| Dibucaine HCl | Dicyclomine HCl |
| Diethylpropion HCl | Dimethisoquin HCl |
| Dimetindene Maleate | Diphemanil Methylsulfate |
| Diphenidol HCl | Diphenoxylate HCl |
| Diphenylpyraline HCl | Dipropyldopamine HBr |
| Dobutamine HCl | Donepezil HCl |
| Doxepin HCl | Droperidol |
| Dyclonine HCl | Ebastine |
| Econazole Nitrate | Epinastine HCl |
| Ethaverine HCl | Ethopropazine HCl |
| Eticlopride HCl, S(-)- | Etofenamate |
| Etonitazenyl Isothiocyanate | Femoxetine HCl |
| Fentanyl Citrate | Fenticonazole Nitrate |
| Fipexide HCl | Flavoxate HCl |
| Flunarizine diHCl | Fluoxetine Related Compound B |
| Fluperlapine | Flurazepam Related Compound C |
| Fluspirilene | Fluvoxamine Maleate |
| GBR 12783 DiHCl | GBR 12909 DiHCl |
| GBR 13069 DiHCl | GBR-12935 DiHCl |
| GR 89696 Fumarate | Guanabenz Acetate |
| Guanadrel Sulfate | Guanethidine Sulfate |
| Halofantrine HCl | Haloperidol |
| HEAT HCl | Hexylcaine HCl |
| Hycanthone | Hydroxychloroquine Sulfate |
| Hydroxyzine HCl | Hyoscyamine Sulfate |
| IBZM, S(-)- | ICI-199,441 HCl |
| Ifenprodil | Imipramine HCl |
| Indatraline HCl | lofetamine HCl |
| Irinotecan HCl | Isamoltane Hemifumarate |
| Isopromethazine HCl | Isoxsuprine HCl |
| Ketanserin L-Tartrate | Ketoconazole |
| Ketotifen Fumarate Salt | L-693,403 Maleate |
| L-741,626 | L-741,742 HCl |
| L-745,870 TriHCl | Labetalol HCl |
| Levetimide HCl, R(-) | Levobunolol HCl |
| Lidoflazine | Lisuride Hydrogen Maleate, R(+)- |
| Lobeline HCl | lomerizine diHCl |
| Loperamide HCl | Loxapine Succinate |
| LY-53,857 Maleate | Mazindol |
| MDL 12,330A HCl | Mebhydroline 1,5-naphthalendisulfonate Salt |
| Meclizine HCl | Mefloquine HCl |
| Meprylcaine HCl | Mesoridazine Besylate |
| Metaphit Methanesulfonate | Metergoline |
| Methantheline Bromide | Methdilazine |
| Methiothepin Mesylate | Methixene HCl |
| METHOCTRAMINE TETRAHYDROCHLORIDE | Methotrimeprazine Maleate |
| Methylene Violet 3Rax HCl | Metipranolol |
| Mianserin HCl | Miconazole |
| ML-9 HCl | Morantel Hydrogen L-Tartrate |
| MR 16728 HCl | N-(2-Chloroethyl)-N-Ethyl-2-Bromobenzylamine HCl |
| N'-[2-(Benzo[1,2,5]Thiadiazole-4-Sulfonylamino)-Acetyl]-Hydrazinecarboxylic Acid 2-(2-{4-[(4-Chloro-Phenyl)-Phenyl-Methyl]-Piperazin-1-Yl}-Ethoxy)-Ethyl Ester | Nafronyl Oxalate Salt |
| Naftifine | Naftopidil diHCl |
| Naltriben Mesylate | NAN-190 HBr |
| NE-100 | Nefazodone |
| Nefopam HCl | Nicardipine HCl |
| Nicergoline | Niguldipine HCl, (+/-)- |
| Nisoxetine HCl | Nylidrin HCl |
| Octoclothepin Maleate, (±)- | Orphenadrine Citrate |
| Oxamniquine | Oxamniquine Related Compound A |
| Oxamniquine Related Compound B | Oxatomide |
| Oxiconazole Nitrate | Oxybutynin HCl |
| Panaxatriol | PAPP |
| Paroxetine | Paxilline |
| p-Chlorobenzhydrylpiperazine | Penbutolol Sulfate |
| Pentamidine Isethionate | Pentazocine, (±)- |
| Pergolide Methanesulfonate | Perhexiline Maleate Salt |
| Perospirone | Perphenazine |
| Perphenazine Sulfoxide | Phenamil Methanesulfonate |
| Phencyclidine HCl | Phenosafranin HCl |
| Phenoxybenzamine HCl | Phenyltoloxamine Citrate Salt |
| Piboserod | Pimozide |
| Pinacyanol Chloride | Pindobind |
| Piperacetazine | Piperazine-1,4-Dicarboxylic Acid Benzyl Ester 2-[4-(4-Dimethylamino-Benzyl)-Piperazin-1-Yl]-Ethyl Ester |
| Piperidolate HCl | Pirenperone |
| PPHT HCl, (±)- | Pramoxine HCl |
| Prenylamine Lactate Salt | Pridinol Methanesulfonate Salt |
| Prochlorperazine Maleate | Procyclidine HCl |
| Proflavine Hemisulfate Salt | Progesterone |
| Promazine HCl | Promethazine HCl |
| Propafenone HCl | Proparacaine HCl |
| Propericyazine | Propiomazine |
| Propranolol HCl | Protokylol |
| Protriptyline HCl | Pyrilamine Maleate |
| Pyrimethamine | Pyrrolidine-1,2-Dicarboxylic Acid 1-[1-(4-Allyloxy-Benzyl)-Piperidin-2-Ylmethyl] Ester 2-Benzyl Ester |
| Pyrvinium Pamoate | Quetiapine Fumarate |
| Quinacrine HCl | Quinaldine Red |
| Quipazine Dimaleate | Quipazine, 6-Nitro-, Maleate |
| Raloxifene | Rimantadine HCl |
| Risperidone | Ritanserin |
| Ritodrine HCl | RS 23597-190 HCl |
| RS 67333 HCl | RS 67506 HCl |
| Safranin O HCl | Salmeterol |
| SB203186 | SCH-23390 HCl, R(+)- |
| Sertaconazole Nitrate | Sertindole |
| Sertraline | Sibutramine HCl |
| SKF-525A HCl | SKF-96365 HCl |
| SNC 121 | Spiperone HCl |
| Sufentanil | T-226296 |
| Tamoxifen Citrate | Tamsulosin HCl |
| Tegaserod Maleate | Terbinafine HCl |
| Terconazole | Terfenadine |
| Terfenadine Related Compound A | Tetracaine HCl |
| Tetrindole Mesylate | Thiethylperazine Malate |
| Thioperamide Maleate | Thioproperazine |
| Thioridazine | Thiothixene |
| Thiothixene, (E)- | Thonzonium Bromide |
| Tiagabine HCl | Tioconazole Related Compound A |
| TMB-8 HCl | Tolterodine L-Tartrate |
| Toremifene Citrate | Tramazoline HCl |
| Trans-U-50488 Methanesulfonate, (±)- | Trazodone HCl |
| Tridihexethyl Chloride | Trifluoperazine HCl |
| Trifluperidol HCl | Triflupromazine HCl |
| Trihexyphenidyl HCl | Trimebutine |
| Trimeprazine Hemi-L-Tartrate | Trimipramine Maleate |
| Tripelennamine HCl | Triprolidine HCl |
| Triprolidine HCl Z Isomer | Tropanyl 3,5-Dimethylbenzoate |
| Tropine 2-(4-Chlorophenoxy)Butanoate, Maleate | U-50488 HCl, (-)- |
| U-62066 | UH 232 Maleate, (+)- |
| Vecuronium Bromide | Verapamil HCl |
| Verapamil Related Compound B | Vesamicol HCl |
| Vinpocetine | W-7 HCl |
| WB-4101 HCl | Xylazine |
| Xylometazoline HCl | |

The term "salt" is to be understood as meaning any form of the active compound according to the invention in which this assumes an ionic form or is charged and is coupled with a counter-ion (a cation or anion) or is in solution. By this are also to be understood complexes of the active compound with other molecules and ions, in particular complexes which are complexed via ionic interactions.

The term "physiologically acceptable salt" is understood in particular, in the context of this invention, as salt (as defined above) formed either with a physiologically tolerated acid, that is to say salts of the particular active compound with inorganic or organic acids which are physiologically tolerated - especially if used on humans and/or mammals - or with at least one, preferably inorganic, cation which are physiologically tolerated - especially if used on humans and/or mammals. Examples of physiologically tolerated salts of particular acids are salts of: hydrochloric acid, hydrobromic acid, sulfuric acid, hydrobromide, monohydrobromide, monohydrochloride or hydrochloride, methiodide, methanesulfonic acid, formic acid, acetic acid, oxalic acid, succinic acid, malic acid, tartaric acid, mandelic acid, fumaric acid, lactic acid, citric acid, glutamic acid, hippuric acid picric acid and/or aspartic acid. Examples of physiologically tolerated salts of particular bases are salts of alkali metals and alkaline earth metals and with NH₄.

The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

The term "treatment", as referred to in the present invention, is defined as the treatment of the symptoms of diabetes-associated pain, the prophylaxis of the symptoms of diabetes-associated pain, as well as the prophylaxis of the disease consequences causing the symptoms of diabetes-associated pain. Preferably "treatment", as referred to in the present invention, is defined as including the treamtent of the symptoms of diabetes-associated pain or the prophylaxis of the symptoms of diabetes-associated pain.

PAIN is defined by the International Association for the Study of Pain (IASP) as "an unpleasant sensory and emotional experience associated with actual or potential tissue damage, or described in terms of such damage (IASP, Classification of chronic pain, 2nd Edition, IASP Press (2002), 210). Even though pain is always subjective its causes or syndromes can be classified.

The term "pain" as used in the present invention exclusively refers to diabetes-associated pain.

"Diabetes-associated pain",as defined in the present invention, preferably includes any form and type of pain/pain syndromes which are related to diabetes. Preferably, said diabetes-associated pain derives from diabetic neuropathy, diabetic retinopathy, diabetic amyotrophy, gastroparesis, diabetic diarrhea, charcot joint, neuropathy of the bladder, diabetic nephropathy and/or optionally diabetic foot problems.

The term "derived from", as defined in the present invention, has the same meaning as the terms "caused by" and/or "associated with", thereby referring to the consequences of the pathological process/es of diabetes which result in pain.

In a preferred embodiment of the invention said diabetic neuropathy preferably comprises autonomic neuropathy, sensorimotoric neuropathy, distal symmetric sensorimotror neuropathy, focal and multifocal neuropathies and/or sensorimotor polyneuropathy.

According to the IASP "allodynia" is defined as "a pain due to a stimulus which does not normally provoke pain" (IASP, Classification of chronic pain, 2^{nd} Edition, IASP Press (2002), 210). Even though the symptoms of allodynia are most likely associated as symptoms of neuropathic pain this is not necessarily the case so that there are symptoms of allodynia not connected to neuropathic pain though rendering allodynia in some areas broader then neuropathic pain.

The IASP draws the following difference between "allodynia", "hyperalgesia" and "hyperpathia" (IASP, Classification of chronic pain, 2nd Edition, IASP Press (2002), 212):

| | | |
|---|---|---|
| Allodynia | Lowered threshold | Stimulus and response mode differ |
| Hyperalgesia | Increased response | Stimulus and response rate are the same |
| Hyperpathia | Raised threshold; Increased response | Stimulus and response rate may be the same or different |

In a preferred embodiment of the invention said diabetes-associated pain is allodynia.

In another aspect of the present invention said allodynia is mechanical allodynia.

In another aspect of the present invention said allodynia is thermal allodynia.

In another preferred embodiment of the invention said diabetes-associated pain is hyperalgesia.

In another preferred embodiment of the invention said diabetes-associated pain is hyperpathia.

According to the IASP "neuropathy" is defined as "a primary lesion or dysfunction in the nervous system" (IASP, Classification of chronic pain, 2nd Edition, IASP Press (2002), 211). Neuropathic pain may have central or peripheral origin.

In a preferred aspect of the present invention said diabetes-associated pain is derived from neuropathy.

In another preferred aspect of the present invention said diabetes-associated pain is derived from peripheral neuropathy.

In a preferred aspect of the present invention said diabetes-associated pain is derived from central neuropathy.

According to the IASP "neuritis" is defined as "Inflammation of a nerve or nerves" (IASP, Classification of chronic pain, 2nd Edition, IASP Press (2002), 212).

In a preferred embodiment of the invention said diabetes-associated pain is neuritis.

According to the IASP "neuralgia" is defined as "Pain in the distribution of a nerve or nerves" (IASP, Classification of chronic pain, 2nd Edition, IASP Press (2002), 212).

In a preferred embodiment of the invention said diabetes-associated pain is neuralgia.

According to the IASP "causalgia" is defined as "a syndrome of sustained burning pain, allodynia and hyperpathia after a traumatic nerve lesion, often combined with vasomotor and sudomotor dysfunction and later trophic changes" (IASP, Classification of chronic pain, 2nd Edition, IASP Press (2002), 210).

In a preferred aspect of the present invention said diabetes-associated pain is causalgia.

In another preferred aspect of the present invention said diabetes-associated pain is preferably derived from diabetic neuropathy, diabetic retinopathy, diabetic amyotrophy, gastroparesis, diabetic diarrhea, charcot joint, neuropathy of the bladder, diabetic nephropathy and/or diabetic foot problems.

In another embodiment of the invention the sigma receptor to which the "compound binding to the sigma receptor" is binding to is the sigma-1 receptor. Under this embodiment "Compound/s binding to the sigma receptor" as used in this application is/are defined as having an IC₅₀ value ≤5000 nM, more preferably ≤1000 nM, more preferably ≤500 nM. More preferably, the IC₅₀ value is ≤250 nM. More preferably, the IC₅₀ value is ≤100 nM. Most preferably, the IC₅₀ value is ≤ 50 nM. Additionally, the wording "Compound/s binding to the sigma receptor", as used in the present application is defined as having at least ≥50% displacement using 10 mM radioligand specific for the sigma receptor (e.g. preferably ¹H-pentazocine) whereby the sigma recepor may be any sigma receptor subtype.

In a highly preferred embodiment of the present invention, the compound binding to the sigma receptor is BD-1047 (N1-(3,4-dichlorophenethyl)-N1,N2,N2-trimethylethane-1,2-diamine).

In a highly preferred embodiment of the present invention, the compound binding to the sigma receptor is BD-1063 (1-(3,4-dichlorophenethyl)-4-methylpiperazine).

In another highly preferred embodiment of the present invention, the compound binding to the sigma-1 receptor is BD-1047 (N1-(3,4-dichlorophenethyl)-N1,N2,N2-trimethylethane-1,2-diamine).

In another highly preferred embodiment of the present invention, the compound binding to the sigma-1 receptor is BD-1063 (1-(3,4-dichlorophenethyl)-4-methylpiperazine).

In a preferred embodiment of the present invention, said compound binding to the sigma receptor has an IC50 value of ≤5000 nM.

In a preferred embodiment of the present invention, said compound binding to the sigma receptor has an IC50 value of ≤1000 nM.

In a preferred embodiment of the present invention, said compound binding to the sigma receptor has an IC50 value of ≤500 nM.

In a preferred embodiment of the present invention, said compound binding to the sigma receptor has an IC50 value of ≤250 nM.

In a preferred embodiment of the present invention, said compound binding to the sigma receptor has an IC50 value of ≤100 nM.

In another preferred embodiment of the present invention, said compound binding to the sigma receptor has an IC50 value of ≤50 nM.

Most preferably, "compounds highly specific for the sigma receptor" are defined as being "Compound/s binding to the sigma receptor", as defined above, having an IC₅₀ value of ≤100 nM.

In a highly preferred embodiment of the present invention, said compounds "highly specific for the sigma receptor" are binding to sigma-1 receptor.

In a highly preferred embodiment of the present invention said compounds are binding to the sigma-1 receptor.

In another possible embodiment of the present invention said compounds are binding to the sigma-2 receptor.

In another preferred embodiment of the invention, said compounds binding to the sigma receptor as defined above, are preferably antagonists, inverse agonists, agonists, partial antagonists and/or partial agonists.

In a possible embodiment of the present invention the compound binding to the sigma receptor as defined above is acting on the sigma receptor as a mixed agonist/antagonist.

In another embodiment of the invention the compound binding to the sigma receptor as defined above is acting on the sigma receptor as an antagonist.

In another embodiment of the invention the compound binding to the sigma receptor as defined above is acting on the sigma receptor-1 as an antagonist.

In another embodiment of the invention the compound binding to the sigma receptor as defined above is acting on the sigma receptor as an inverse agonist.

In another embodiment of the invention the compound binding to the sigma receptor as defined above is acting on the sigma receptor as a partial antagonist.

In another possible embodiment of the invention the compound binding to the sigma receptor as defined above is acting on the sigma receptor as an agonist.

A further aspect of the present invention relates to a medicament in different pharmaceutical forms comprising at least a compound binding to the sigma receptor and optionally at least one further active substance and/or optionally at least one auxiliary substance.

Preferably, the medicament is suitable for oral or parenteral administration, more preferably for oral, intravenous, intraperitoneal, intramuscular, subcutaneous, intrathekal, rectal, transdermal, transmucosal or nasal administration.

Medicaments for oral administration are preferably selected from the group consisting of tablets, drageés, capsules, powders, drops, gels, juices, sirups, solutions and suspensions.

The medicament of the present invention for oral administration may also be in the form of multiparticulates, preferably microparticles, microtablets, pellets or granules, optionally compressed into a tablet, filled into a capsule or suspended in a suitable liquid. Suitable liquids are known to those skilled in the art. The respective medicament may - depending on its route of administration - also contain one or more auxiliary substances known to those skilled in the art. The medicament according to the present invention may be produced according to standard procedures known to those skilled in the art.

The daily dosage for humans and animals may vary depending on factors that have their basis in the respective species or other factors, such as age, sex, weight or degree of illness and so forth. The daily dosage for humans may preferably be in the range from1 to 2000, preferably 1 to 1500, more preferably 1 to 1000 milligrams of active substance to be administered during one or several intakes per day.

### Pharmacological Methods

### Protocol for streptozotocin (STZ)-Induced Diabetes

The pancreatic β-cell cytotoxic agent STZ is widely used to induce diabetes in rodents. The glucosamine-nitrosourea compound STZ is taken up into the insulin-producing β-cells of the islets of Langerhan's via the GLUT-2 glucose transporter. The cytotoxic effect of STZ is mediated through a decrease in NAD levels, and the formation of intracellular free radicals leading to various toxic effects, including DNA-strand breaks (Schnedl et al., 1994). The STZ-induced diabetic rodents are hypoinsulinemic, but generally do not require exogenous insulin treatment to survive during the first days after STZ-induced diabetes. STZ-induced diabetic rodents show common features of human diabetes that include damage to the eye, kidney, blood vessels, and nervous system. Diabetic neuropathic pain occurs mainly due to the damage in the nervous system (Sima and Sugimoto, 1999).

**Method:** In the present study, diabetes is induced in mice by a single i.v. injection of STZ (200 mg/kg) as reported previously (Kamei et al., 1991; Rashid and Ueda, 2002). Mice weighing -30 g are injected i.v. with STZ in the tail vein. STZ solution is prepared fresh by dissolving it in saline adjusted to pH 4.5 in 0.1 N citrate buffer. Age-matched non-diabetic control mice are injected with the vehicle alone. Due to frequent urination (polyuria) in the diabetic mice, special care is needed for these animals. The STZ-injected mice are kept in a group of four per cage. The bed of the cage is changed daily and special attention is paid to food and water supplement. The plasma glucose level in the mice is measured in blood samples obtained from tail vein. Only mice with a plasma glucose concentration greater than 300 mg/dl (16.7 mM) are considered as diabetic. All efforts are made to minimize both the sufferings and number of animals used.

Thermal hyperalgesia and mechanical allodynia is measured at different time points (1-21 days) after STZ injection. Mechanical allodynia is measured using von-Frey filaments, a well-established method known by those skilled in the art, where the paw withdrawal response is measured. Similarly, thermal hyperalgesia is measured as the latency to paw withdrawal evoked by exposing the right hind paw to a thermal stimulus.

### Von Frey-Test

The von Frey model is a model for allodynia, stimulated mechanically and known by persons skilled in the art.

The examples in the following section describing pharmacological trials are merely illustrative and the invention cannot be considered in any way as being restricted to these applications.

### Examples

### Example 1: Effect of BD-1063 on mechanical allodynia in STZ-induced mice

BD-1063 is a well known compound with high affinity to the sigma receptor, as disclosed in WO99/59409. This pharmacological test shows the effect of BD-1063 (IC₅₀=30 nM sigma-1/800 nM sigma-2), a specific sigma receptor inhibitor, in the von-Frey test.

It can be seen that BD-1063 significantly antagonizes mechanical allodynia in the von-Frey test.

## Claims

1. Use of at least one compound binding to the sigma receptor for the production of a medicament for the treatment of diabetes-associated pain.

2. Use, according to claim 1, **characterized in that** said compound binding to the sigma receptor may be in neutral form, the form of a base or acid, in the form of a salt, preferably a physiologically acceptable salt, in the form of a solvate or of a polymorph and/or in the form of in the form of its racemate, pure stereoisomers, especially enantiomers or diastereomers or in the form of mixtures of stereoisomers, especially enantiomers or diastereomers, and/or in any mixing ratio.

3. Use, according to claim 1, **characterized in that** said compound binding to the sigma receptor has an IC₅₀ value of ≤5000 nM.

4. Use, according to claim 1, **characterized in that** said compound binding to the sigma receptor has an IC₅₀ value of ≤1000 nM.

5. Use, according to claim 1, **characterized in that** said compound binding to the sigma receptor has an IC₅₀ value of ≤500 nM.

6. Use, according to claim 1, **characterized in that** said compound binding to the sigma receptor has an IC₅₀ value of ≤250 nM.

7. Use, according to claim 1, **characterized in that** said compound binding to the sigma receptor has an IC₅₀ value of ≤100 nM.

8. Use, according to claim 1, **characterized in that** said compound binding to the sigma receptor has an IC₅₀ value of ≤50 nM.

9. Use, according to claim 1, **characterized in that** said compound binding to the sigma receptor is selected from the group consisting of:
| | |
|---|---|
| (-)-Cyanopindolol hemifumarate | (-)-SPARTEINE SULFATE PENTAHYDRATE |
| (+)-HIMBACINE | (2-Dibutylamino-Ethyl)-Carbamic Acid 2-(4-Benzofuran-2-Ylmethyl-Piperazin-1-Yl)-Ethyl Ester |
| (4-[1,2,3]Thiadiazol-4-Yl-Benzyl)-Carbamic Acid 1-(3-Methoxy-2-Nitro-Benzyl)-Piperidin-3-Ylmethyl Ester | (S)-Methamphetamine HCl |
| [1-(9-Ethyl-9H-Carbazol-3-Ylmethyl)-Pyrrolidin-3-Yl]-Carbamic Acid 1-(3-Benzyloxy-4-Methoxy-Benzyl)-Piperidin- 3-Ylmethyl Ester | [1-(9-Ethyl-9H-Carbazol-3-Ylmethyl)-Pyrrolidin-3-Yl]-Carbamic Acid 2-(Tert-Butoxycarbonyl-Naphthalen-1-Ylmethyl-Amino)-Ethyl Ester |
| [4-(4-Ethyl-3.5-Dimethyl-Pyrazol-1-Yl)-Phenyl]-[4-(3-Phenyl-Allyl)-Piperazin-1-Yl]-Methanone | 1-(1,2-Diphenylethyl)Piperidine Maleate, (+/-) |
| 1-(1-Naphthyl)Piperazine HCl | 1-(3-Chlorophenyl)Piperazine HCl |
| 1-(4-Bromo-Benzenesulfonyl)-4-(2-Tert-Butylsulfanyl-Benzyl)-Piperazine | 2-(2-{[1-(3-Chloro-Benzyl)-Pyrrolidin-3-Yl]-Methyl-Carbamoyl}-2-Methyl-Propyl)-4,6-Dimethyl-Benzoic Acid |
| 2-Chloro-11-(4-Methylpiperazino)Dibenz[B,F]Oxepin Maleate | 3,3'-Diethylthiacarbocyanine lodide |
| 3-Mercapto-2-Methylpropanoic Acid 1,2-Diphenylethylamine Salt | 3-Quinuclidinyl Benzilate |
| 3-Tropanyl-3,5-Dichlorobenzoate | 3-Tropanyl-Indole-3-Carboxylate HCl |
| 4-(1H-Indol-4-Yl)-Piperazine-1-Carboxylic Acid 2-(5-Bromo-2-Ethoxy-Phenylamino)-Cyclohexylmethyl Ester | 4-(2-Tert-Butylsulfanyl-Benzyl)-Piperazine-1-Carboxylic Acid 2-Thiophen-2-Yl-Ethyl Ester |
| 4-(3,5-Dimethoxy-Phenyl)-Piperazine-1-Carboxylic Acid 1-(2-Fluoro-Benzyl)-Piperidin-2-Ylmethyl Ester | 4-(3-Nitro-5-Sulfamoyl-Thiophen-2-Yl)-Piperazine-1-Carboxylic Acid 1-(2-Fluoro-5-Methoxy-Benzyl)-Piperidin-3-Ylmethyl Ester |
| 4-(4-Fluorobenzoyl)-1-(4-Phenylbutyl)Piperidine Oxalate | 4-(5-Trifluoromethyl-Pyridin-2-Yl)-Piperazine-1-Carboxylic Acid Pent-2-Ynyl Ester |
| 4,4'-Bis[4-(P-Chlorophenyl)-4-Hydroxypiperidino]Butyrophenone | 4-[1-(4-Chlorobenzyl)-4-(benzylpiperidin-4-yl]-2-hydroxy-4-oxobut-2-enoic acid |
| 4-Bromo-N-[1-(9-Ethyl-9H-Carbazol-3-Ylmethyl)-Pyrrolidin-3-Yl]-2-Trifluoromethoxy-Benzenesulfonamide | 4'-Chloro-3-Alpha-(Diphenylmethoxy)Tropane HCl |
| 4-Furan-2-Ylmethyl-Piperazine-1-Carboxylic Acid 2-{4-[3-(2-Trifluoromethyl-Phenothiazin-10-Yl)-Propyl]-Piperazin-1-Yl}-Ethyl Ester | 4-Methoxy-N-[1-(7-Methoxy-Benzo[1,3]Dioxol-5-Ylmethyl)-Pyrrolidin-3-Yl]-Benzenesulfonamide |
| 5-(N-Ethyl-N-Isopropyl)-Amiloride | 7-Hydroxy-DPAT HBr, (±)- |
| 8-Hydroxy-DPAT HBr, (R)-(+)- | 8-Hydroxy-DPAT HBr, S(-)- |
| 9-[4-({[4'-(trifluoromethyl)-1,1'-biphenyl-2-yl]carbonyl}amino)piperidin-1-yl]-N-(2,2,2-trifluoroethyl)-9H-fluorene-9-carboxamide | Acepromazine Maleate |
| Acetophenazine Maleate | Acrinol |
| Ajmaline | Alaproclate HCl |
| Aloe-Emodin | Alprenolol D-Tartrate Salt Hydrate |
| Alprenolol HCl | AMI-193 |
| Aminobenztropine | Amiodarone HCl |
| Amodiaquine HCl | Amorolfine HCl |
| Amoxapine | Anileridine HCl |
| Anisotropine Methylbromide | Anpirtoline |
| ARC 239 DiHCl | Astemizole |
| Auramine O HCl | Azaperone |
| Azatadine Maleate | Azelastine HCl |
| Bamethan sulfate | BD 1008 DiHBr |
| BD-1047 (N1-(3,4-dichlorophenethyl)-N1,N2,N2-trimethylethane-1,2-diamine) | BD-1063 (1-(3,4-dichlorophenethyl)-4-methylpiperazine) |
| Benextramine TetraHCl | Benfluorex HCl |
| Benidipine HCl | Benoxathian HCl |
| Benoxinate HCl | Benperidol |
| Benproperine Phosphate | Benzododecinium bromide |
| Benzphetamine HCl | Benztropine Mesylate |
| Benzydamine HCl | Bephenium Hydroxynaphthoate |
| Bepridil HCl | Berberine chloride |
| Betaxolol HCl | Bifemelane |
| BMY 7378 DiHCl | Bopindolol Malonate |
| BP 554 Maleate | Bromhexine HCl |
| Bromodiphenhydramine HCl | Bromperidol |
| Brompheniramine Maleate | BTCP HCl |
| Buclizine HCl | Buflomedil HCl |
| Bupropion HCl | Buspirone HCl |
| Butacaine Sulfate | Butaclamol HCl, (±)- |
| Butenafine HCl | Butoconazole Nitrate |
| BW 723C86 HCl | Carbetapentane Citrate |
| Carbinoxamine Maleate | Carpipramine DiHCl DiH2O |
| Carvedilol | Cephapirin Benzathine |
| CGS-12066A Maleate | Chloroprocaine HCl |
| Chloroquine Phosphate | Chlorpheniramine Maleate |
| Chlorphenoxamine HCl | Chlorpromazine HCl |
| Chlorprothixene | Cinanserin HCl |
| Cinnarizine | Cirazoline HCl |
| Cis-(+/-)-N-Methyl-N-[2-(3,4-Dichlorophenyl)Ethyl]-2-(1-Pyrrolidinyl)Cyclohexamine DiHBr | Cis(Z)-Flupentixol DiHCl |
| Cisapride Hydrate | Citalopram HBr |
| Clebopride Maleate Salt | Clemastine Fumarate |
| Clemizole HCl | Clenbuterol HCl |
| Clidinium Bromide | Clobenpropit 2HBr |
| Clofazimine | Clofilium Tosylate |
| Clomiphene Citrate | Clomiphene Related Compound A |
| Cloperastine HCl | Clorgyline HCl |
| Clozapine | CONESSINE |
| Cyclizine | Cyclobenzaprine HCl |
| Cycloheximide | Cyproheptadine HCl |
| Darrow Red HCl | Demecarium Bromide |
| Denatonium Benzoate | Deptropine Citrate |
| Desloratadine | Dexbrompheniramine Maleate |
| Dexchlorpheniramine Maleate | Dexfenfluramine HCl |
| Dibucaine HCl | Dicyclomine HCl |
| Diethylpropion HCl | Dimethisoquin HCl |
| Dimetindene Maleate | Diphemanil Methylsulfate |
| Diphenidol HCl | Diphenoxylate HCl |
| Diphenylpyraline HCl | Dipropyldopamine HBr |
| Dobutamine HCl | Donepezil HCl |
| Doxepin HCl | Droperidol |
| Dyclonine HCl | Ebastine |
| Econazole Nitrate | Epinastine HCl |
| Ethaverine HCl | Ethopropazine HCl |
| Eticlopride HCl, S(-)- | Etofenamate |
| Etonitazenyl Isothiocyanate | Femoxetine HCl |
| Fentanyl Citrate | Fenticonazole Nitrate |
| Fipexide HCl | Flavoxate HCl |
| Flunarizine diHCl | Fluoxetine Related Compound B |
| Fluperlapine | Flurazepam Related Compound C |
| Fluspirilene | Fluvoxamine Maleate |
| GBR 12783 DiHCl | GBR 12909 DiHCl |
| GBR 13069 DiHCl | GBR-12935 DiHCl |
| GR 89696 Fumarate | Guanabenz Acetate |
| Guanadrel Sulfate | Guanethidine Sulfate |
| Halofantrine HCl | Haloperidol |
| HEAT HCl | Hexylcaine HCl |
| Hycanthone | Hydroxychloroquine Sulfate |
| Hydroxyzine HCl | Hyoscyamine Sulfate |
| IBZM, S(-)- | ICI-199,441 HCl |
| Ifenprodil | Imipramine HCl |
| Indatraline HCl | lofetamine HCl |
| Irinotecan HCl | Isamoltane Hemifumarate |
| Isopromethazine HCl | Isoxsuprine HCl |
| Ketanserin L-Tartrate | Ketoconazole |
| Ketotifen Fumarate Salt | L-693,403 Maleate |
| L-741, 626 | L-741,742 HCl |
| L-745,870 TriHCl | Labetalol HCl |
| Levetimide HCl, R(-) | Levobunolol HCl |
| Lidoflazine | Lisuride Hydrogen Maleate, R(+)- |
| Lobeline HCl | lomerizine diHCl |
| Loperamide HCl | Loxapine Succinate |
| LY-53,857 Maleate | Mazindol |
| MDL 12,330A HCl | Mebhydroline 1,5-naphthalendisulfonate Salt |
| Meclizine HCl | Mefloquine HCl |
| Meprylcaine HCl | Mesoridazine Besylate |
| Metaphit Methanesulfonate | Metergoline |
| Methantheline Bromide | Methdilazine |
| Methiothepin Mesylate | Methixene HCl |
| METHOCTRAMINE TETRAHYDROCHLORIDE | Methotrimeprazine Maleate |
| Methylene Violet 3Rax HCl | Metipranolol |
| Mianserin HCl | Miconazole |
| ML-9 HCl | Morantel Hydrogen L-Tartrate |
| MR 16728 HCl | N-(2-Chloroethyl)-N-Ethyl-2-Bromobenzylamine HCl |
| N'-[2-(Benzo[1,2,5]Thiadiazole-4-Sulfonylamino)-Acetyl]-Hydrazinecarboxylic Acid 2-(2-{4-[(4-Chloro-Phenyl)-Phenyl-Methyl]-Piperazin-1-Yl}-Ethoxy)-Ethyl Ester | Nafronyl Oxalate Salt |
| Naftifine | Naftopidil diHCl |
| Naltriben Mesylate | NAN-190 HBr |
| NE-100 | Nefazodone |
| Nefopam HCl | Nicardipine HCl |
| Nicergoline | Niguldipine HCl, (+/-)- |
| Nisoxetine HCl | Nylidrin HCl |
| Octoclothepin Maleate, (±)- | Orphenadrine Citrate |
| Oxamniquine | Oxamniquine Related Compound A |
| Oxamniquine Related Compound B | Oxatomide |
| Oxiconazole Nitrate | Oxybutynin HCl |
| Panaxatriol | PAPP |
| Paroxetine | Paxilline |
| p-Chlorobenzhydrylpiperazine | Penbutolol Sulfate |
| Pentamidine Isethionate | Pentazocine, (±)- |
| Pergolide Methanesulfonate | Perhexiline Maleate Salt |
| Perospirone | Perphenazine |
| Perphenazine Sulfoxide | Phenamil Methanesulfonate |
| Phencyclidine HCl | Phenosafranin HCl |
| Phenoxybenzamine HCl | Phenyltoloxamine Citrate Salt |
| Piboserod | Pimozide |
| Pinacyanol Chloride | Pindobind |
| Piperacetazine | Piperazine-1,4-Dicarboxylic Acid Benzyl Ester 2-[4-(4-Dimethylamino-Benzyl)-Piperazin-1-Yl]-Ethyl Ester |
| Piperidolate HCl | Pirenperone |
| PPHT HCl, (±)- | Pramoxine HCl |
| Prenylamine Lactate Salt | Pridinol Methanesulfonate Salt |
| Prochlorperazine Maleate | Procyclidine HCl |
| Proflavine Hemisulfate Salt | Progesterone |
| Promazine HCl | Promethazine HCl |
| Propafenone HCl | Proparacaine HCl |
| Propericyazine | Propiomazine |
| Propranolol HCl | Protokylol |
| Protriptyline HCl | Pyrilamine Maleate |
| Pyrimethamine | Pyrrolidine-1,2-Dicarboxylic Acid 1-[1-(4-Allyloxy-Benzyl)-Piperidin-2-Ylmethyl] Ester 2-Benzyl Ester |
| Pyrvinium Pamoate | Quetiapine Fumarate |
| Quinacrine HCl | Quinaldine Red |
| Quipazine Dimaleate | Quipazine, 6-Nitro-, Maleate |
| Raloxifene | Rimantadine HCl |
| Risperidone | Ritanserin |
| Ritodrine HCl | RS 23597-190 HCl |
| RS 67333 HCl | RS 67506 HCl |
| Safranin O HCl | Salmeterol |
| SB203186 | SCH-23390 HCl, R(+)- |
| Sertaconazole Nitrate | Sertindole |
| Sertraline | Sibutramine HCl |
| SKF-525A HCl | SKF-96365 HCl |
| SNC 121 | Spiperone HCl |
| Sufentanil | T-226296 |
| Tamoxifen Citrate | Tamsulosin HCl |
| Tegaserod Maleate | Terbinafine HCl |
| Terconazole | Terfenadine |
| Terfenadine Related Compound A | Tetracaine HCl |
| Tetrindole Mesylate | Thiethylperazine Malate |
| Thioperamide Maleate | Thioproperazine |
| Thioridazine | Thiothixene |
| Thiothixene, (E)- | Thonzonium Bromide |
| Tiagabine HCl | Tioconazole Related Compound A |
| TMB-8 HCl | Tolterodine L-Tartrate |
| Toremifene Citrate | Tramazoline HCl |
| Trans-U-50488 Methanesulfonate, (±)- | Trazodone HCl |
| Tridihexethyl Chloride | Trifluoperazine HCl |
| Trifluperidol HCl | Triflupromazine HCl |
| Trihexyphenidyl HCl | Trimebutine |
| Trimeprazine Hemi-L-Tartrate | Trimipramine Maleate |
| Tripelennamine HCl | Triprolidine HCl |
| Triprolidine HCl Z Isomer | Tropanyl 3,5-Dimethylbenzoate |
| Tropine 2-(4-Chlorophenoxy)Butanoate, Maleate | U-50488 HCl, (-)- |
| U-62066 | UH 232 Maleate, (+)- |
| Vecuronium Bromide | Verapamil HCl |
| Verapamil Related Compound B | Vesamicol HCl |
| Vinpocetine | W-7 HCl |
| WB-4101 HCl | Xylazine |
| Xylometazoline HCl | |

10. Use according to any of claims 1 to 9, **characterized in that** said compound binding to the sigma receptor is BD-1063/1-(3,4-dichlorophenethyl)-4-methylpiperazine.

11. Use according to any of claims 1 to 9, **characterized in that** said compound binding to the sigma receptor is BD-1047/N1-(3,4-dichlorophenethyl)-N1,N2,N2-trimethylethane-1,2-diamine.

12. Use according to any of claims 1 to 11, **characterized in that** said diabetes-associated pain is central pain.

13. Use according to any of claims 1 to 11, **characterized in that** said diabetes-associated pain is peripheral pain.

14. Use according to any of claims 1 to 11, **characterized in that** said diabetes-associated pain is allodynia.

15. Use according to any of claims 1 to 3, and 9 to 11, **characterized in that** said diabetes-associated pain is mechanical allodynia.

16. Use according to any of claims 1 to 3 and 9 to 11, **characterized in that** said diabetes-associated pain is thermal allodynia.

17. Use according to any of claims 1 to 3 and 9 to 11, **characterized in that** said diabetes-associated pain is hyperalgesia.

18. Use according to any of claims 1 to 3 and 9 to 11, **characterized in that** said diabetes-associated pain is hyperpathia.

19. Use according to any of claims 1 to 3 and 9 to 11, **characterized in that** said diabetes-associated pain is derived from neuropathy.

20. Use according to any of claims 1 to 3 and 9 to 11, **characterized in that** said diabetes-associated pain is derived from peripheral neuropathy.

21. Use according to any of claims 1 to 3 and 9 to 11, **characterized in that** said diabetes-associated pain is derived from central neuropathy.

22. Use according to any of claims 1 to 3 and 9 to 11, **characterized in that** said diabetes-associated pain is neuritis

23. Use according to any of claims 1 to 3 and 9 to 11, **characterized in that** said diabetes-associated pain is neuralgia.

24. Use according to any of claims 1 to 3 and 9 to 11, **characterized in that** said diabetes-associated pain is causalgia.

25. Use according to any of claims 1 to 3 and 9 to 11, **characterized in that** said diabetes-associated pain is preferably derived from diabetic neuropathy, diabetic retinopathy, diabetic amyotrophy, gastroparesis, diabetic diarrhea, charcot joint, neuropathy of the bladder, diabetic nephropathy and/or diabetic foot problems.

26. Use according to any of claims 1 to 25, **characterized in that** said compound binding to the sigma receptor are binding to the sigma-1 receptor.

27. Use according to any of claims 1 to 26, **characterized in that** said compound is acting as an antagonist.

28. Use, according to any of claims 1 to 26, **characterized in that** said compound is acting as an antagonist on the sigma-1 receptor.

29. Use according to any of claims 1 to 26, **characterized in that** said compound is acting as a partial antagonist.

30. Use according to any of claims 1 to 26, **characterized in that** said compound is acting as an inverse agonist.
